(19) Europäisches Patentamt
European Patent Office
Office européen des brevets

(11) Veröffentlichungsnummer: **0 326 936 A2**

(12) # EUROPÄISCHE PATENTANMELDUNG

(21) Anmeldenummer: 89101314.6

(51) Int. Cl.⁴: A61K 31/415

(22) Anmeldetag: 26.01.89

(30) Priorität: 30.01.88 DE 3802776
09.02.88 DE 3803842

(43) Veröffentlichungstag der Anmeldung:
09.08.89 Patentblatt 89/32

(84) Benannte Vertragsstaaten:
AT BE CH DE ES FR GB GR IT LI LU NL SE

(71) Anmelder: BOEHRINGER INGELHEIM KG
Postfach 200
D-6507 Ingelheim am Rhein(DE)

(84) BE CH DE ES FR GR IT LI LU NL SE AT

Anmelder: BOEHRINGER INGELHEIM INTERNATIONAL G.M.B.H.
Postfach 20
D-6507 Ingelheim am Rhein(DE)

(84) GB

(72) Erfinder: Reichl, Richard, Dr.
Pfarrer-Heberer-Strasse 37
D-6530 Bingen(DE)
Erfinder: Walland, Alexander, Dr.
Wilhelm-Leuschner-Strasse 20
D-6507 Ingelheim am Rhein(DE)
Erfinder: Hergenröder, Stefan
Am Stollhenn 41
D-6500 Mainz-Mombach(DE)

(54) **Verwendung von 2-(Phenylamino)-2-imidazolinen zur Anwendung in der Schocktherapie.**

(57) Die Erfindung betrifft die Verwendung von 2-(Phenylamino)-2-imidazolinen zur akuten Schocktherapie.

EP 0 326 936 A2

## Verwendung von 2-(Phenylamino)-2-imidazolinen zur Anwendung in der Schocktherapie

Die Erfindung betrifft die Verwendung von 2-(Phenylamino)-2-imidazolinen zur Anwendung in der Schocktherapie.

Es ist bekannt, daß Phenylaminoimidazoline und deren Säureadditionssalze wertvolle pharmakologische Eigenschaften besitzen. Die Verbindungen, ihre Herstellung und Verarbeitung zu pharmazeutischen Anwendungsformen sind beispielsweise in den deutschen Offenlegungsschriften 13 03 141, 19 58 201 und 26 36 732 beschrieben, wobei die analgetischen, blutdrucksenkenden oder bradikarden Eigenschaften der in diesen Offenlegungsschriften offenbarten Verbindungen herausgestellt werden.

Überraschenderweise wurde gefunden, daß Imidazoline der allgemeinen Formel I

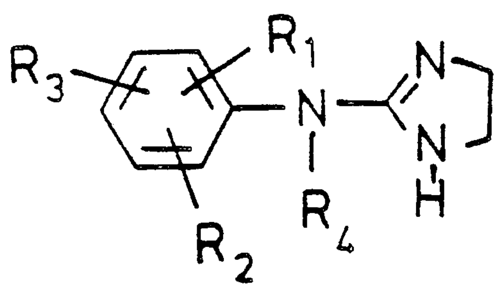

worin

$R_1$ $R_2$ und $R_3$, die gleich oder verschieden sein können, Wasserstoff, Halogen, Hydroxy, $C_1$ bis $C_4$ Alkyl, $C_1$ bis $C_4$ - Halogenalkyl oder $C_1$-$C_4$ Alkoxy und

$R_4$ Wasserstoff, $CH_2-CH=CHR_5$ oder $CH_2-C\equiv C-R_5$ worin

$R_5$ Wasserstoff, Halogen, Methyl oder Ethyl bedeuten können sowie gegebenenfalls deren pharmakologisch verträgliche Säureadditionssalze zur Anwendung in der Schocktherapie, insbesondere beim hämorrhagischen Schock, aber auch beim Endotoxin-Schock und traumatischen Schock besonders geeignet sind. Bevorzugt sind Verbindungen der allgemeinen Formel I, worin die Reste $R_1$ und $R_2$ in ortho Position stehen und Chlor, Brom, Methyl und/oder Ethyl, $R_3$ in 4-Position Wasserstoff oder Methyl und $R_4$ Wasserstoff bedeuten. Weiterhin bevorzugt sind Verbindungen worin $R_1$ Wasserstoff, $R_2$ in 3-Position Halogen - bevorzugt Fluor - und $R_3$ in 4-Position Methyl oder Ethylung und $R_4$ Wasserstoff bedeuten. Im Fall von $R_4$ gleich Allyl oder Methylpropargyl sind bevorzugte Reste $R_1$ und $R_2$ in ortho Position Chlor, Brom, Methyl und/oder Ethyl und $R_3$ Wasserstoff.

Als Alkylgruppen werden - auch als Substituenten andere Reste - beispielsweise Methyl, Ethyl, Propyl, iso-Propyl, Butyl, iso-Butyl, sec-Butyl und tert.-Butyl verstanden. Halogen bedeutet einen Rest aus der Gruppe Fluor, Chlor, Brom oder Jod - bevorzugt Chlor und Brom.

Besonders bevorzugt sind

2-[2,6-Diethylphenylimino]imidazolidin (DE-OS 1303 141),
2-[N-2,6-Dichlorphenyl-N-2-butinylamino]imidazolin-(2) (DE-OS 35 14 351),
2-[N-2,6-Dichlorphenyl-N-allylamino]imidazolin (DE-OS 19 58 201). Aus der deutschen Offenlegungsschrift 28 31 190 ist die Verwendung von

2-[N-2,6-Dichlorphenyl-N-allylamino]imidazolin-(2) sowie dessen Säueadditionssalze als bradykardes Mittel bekannt. Ferner sind aus der DE-OS 36 20 433 die cytoprotectiven Eigenschaften bekannt. Die Verbindung ist in der Literatur auch unter dem Namen Alinidin bekannt.

Die Eignung von Alinidin und anderen Verbindungen der allgemeinen Formel I zur erfindungsgemäßen Anwendung bei der Schocktherapie wurde in tierexperimentellen Untersuchungen an einem Cremaster-Modell bestätigt. Dieses Modell stellt eine etablierte Methode dar, (S. Hergenröder "Mikrozirkulation im hämorrhagischen Schock", Diplomarbeit am Fachbereich Biologie der Joh.-Gutenberg-Universität, Mainz 1987). An diesem Modell werden Einflüsse auf den systemischen Blutdruck, die Herzfrequenz, den daraus errechneten Schockindex sowie wichtige mikrozirkulatorische Parameter, wie Arteriolendurchmesser, die Eryhthrozytenfließgeschwindigkeit und die daraus errechnete Mikroperfusion (Organdurchblutung) und die

Überlebensrate bestimmt. Der Versuchsablauf besteht aus einer präparativen Kontrollphase, gefolgt von einer spontanen Entblutung mit einem raschen Blutdruckabfall auf 25 mm Hg, einer isovolämischen intravenösen 10-minütigen Infusion mit physiologischer NaCl-Lösung (Kontrolltiere) bzw. physiologischer NaCl-Lösung mit darin gelösten Wirkstoffen und einer abschließenden Nachbeobachtungsphase (60 - 120 Minuten). Das zweite Modell unterscheidet sich durch eine zweitägige Nachbeobachtungsphase ohne Präparation des M. cremaster.

Das Ende der Entblutungsphase ist gekennzeichnet durch einen Stopflow im intravital beobachteten mikrozirkulären Areal, bedingt durch den drastischen Blutverlust und dem damit verbundenen starken Blutdruckabfall.

Die hämodynamischen Effekte in der mit physiologischer NaCl-Lösung behandelten Kontrollgruppe waren gering und nur von kurzer Dauer mit einer Überlebensrate von nur 4 von 20 Tieren nach 60 Minuten.

In der mit Alinidin (1 mg/kg) behandelten Gruppe (N = 6) wurde dagegen eine Erholung der systemischen, insbesondere der mikrozirkulatorischen Parameter festgestellt. Es ergab sich eine Überlebensrate von 100 %.

Im zweiten "Schock-Modell" zeigt sich eine ähnlich hohe Überlebensrate der mit der erfindungsgemäß vorgeschlagenen Verbindung behandelten Gruppe.

Während in der nicht vorbehandelten Gruppe 3 von 15 Tieren starben, überlebten in der mit Alinidin vorbehandelten Gruppe 13 von 15 Tieren. Weitere Ergebnisse sind in der nachfolgenden Tabelle zusammengefaßt.

Tabelle I

| Verbindung | Anzahl (N) | Überlebende |
|---|---|---|
| A | 15 | 10 |
| B | 15 | 10 |
| C | 15 | 13 |
| Kontrolle | 40 | 12 |

A = 2,6-Diethylphenylimino]imidazolidin
B = N-2,6-Dichlorphenyl-N-2-butinylamino-]imidazolin-(2)
C = Alinidin

Aufgrund der pharmakologischen Befunde sind die Verbindungen der allgemeinen Formel I sowie ihre pharmakologisch verträglichen Säureadditionssalze zur Verwendung in der Schocktherapie geeignet.

Zur akuten Schockbehandlung können die Verbindungen intravenös - als Bolus-Injektion oder als Infusion -appliziert werden. Der therapeutisch wirksame Dosisbereich liegt zwischen 0.1 und 10 mg/kg.

Beispiele

Herstellungsbeispiel für den Wirkstoff (A)

2-[N-(2,6-Dichlorohenyl)-N-allyl-amino]-imidazolin-(2)

2,0 g 2-(2,6-Dichlorphenylamino)-2-imidazolin werden zusammen mit 3 ml Allylbromid und 1 ml Pyridin in 10 ccm absolutem Methanol ca. 15 Stunden lang im Rohr auf 100 °C erwärmt. Die Reaktionsmischung wird hierauf im Vakuum zur Trockene eingeengt und der verbleibende Rückstand in wenig verdünnter Salzsäure gelöst. Zu ihrer Reinigung wird die salzsaure Lösung mit Äther extrahiert und die Ätherextrakte verworfen. Die hierauf mit 5 n Natronlauge freigesetzte ölige Imidazolinbase kristallisiert nach einiger Zeit unter Eiskühlung durch. Sie wird abgesaugt, mit destilliertem Wasser gewaschen und getrocknet. Die Ausbeute beträgt 1,5 g, das sind 83,8 % der Theorie.

Fp. 130 - 131 °C. Das auf übliche Weise hergestellte Nitrat schmilzt bei 136 - 138 °C. Andere Säureadditionssalze lassen sich nach bekannten Verfahren herstellen.

Beispiel B: Ampullen

2-[N-2,6-Dichlorphenyl-N-allylamino]imidazolin-(2)-

| | |
|---|---|
| HBr | 1,0 mg |
| Natriumchlorid | 18,0 mg |
| dest. Wasser | ad 2,0 ml |

Herstellung:

Wirkstoff und Natriumchlorid werden in Wasser gelöst und unter Stickstoff in Glasampullen abgefüllt.

**Ansprüche**

1) Verwendung von Imidazolinen der allgemeinen Formel I

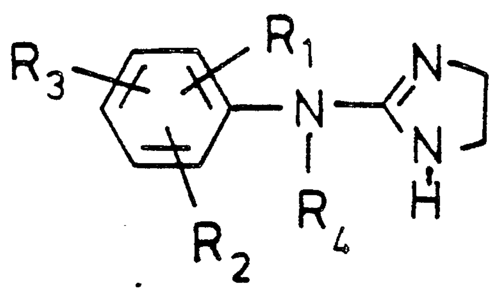

worin
$R_1$ $R_2$ und $R_3$, die gleich oder verschieden sein können, Wasserstoff, Halogen, Hydroxy, $C_1$ bis $C_4$ Alkyl, $C_1$ bis $C_4$ - Halogenalkyl oder $C_1$-$C_4$ Alkoxy und
$R_4$ Wasserstoff, $CH_2$-CH = $CHR_5$ oder $CH_2$-C≡C-$R_5$ worin
$R_5$ Wasserstoff, Halogen, Methyl oder Ethyl bedeuten können sowie gegebenenfalls deren pharmakologisch verträgliche Säureadditionssalze zur Herstellung eines Arzneimittels zur Anwendung in der Schocktherapie.

2) Verwendung von Verbindungen der allgemeinen Formel I gemäß Anspruch 1, worin die Reste $R_1$ und $R_2$ in ortho Position stehen und Chlor, Brom, Methyl und/oder Ethyl, $R_3$ in 4-Position Wasserstoff oder Methyl und $R_4$ Wasserstoff bedeuten, oder worin $R_1$ Wasserstoff, $R_2$ in 3-Position Halogen -bevorzugt Fluor - und $R_3$ in 4-Position Methyl oder Ethylung und $R_4$ Wasserstoff bedeuten, oder $R_4$ gleich Allyl oder Methylpropargyl die Reste $R_1$ und $R_2$ in ortho Position Chlor, Brom, Methyl und/oder Ethyl und $R_3$ Wasserstoff bedeuten, sowie deren Säureadditionssalze zur Herstellung eines Arzneimittels zur Anwendung in der Schocktherapie.

3) Verwendung von 2-[N-2,6-Dichlorphenyl-N-allylamino]-2-imidazolin und dessen Säureadditionssalze zur Herstellung eines Arzneimittels zur Anwendung in der Schocktherapie.